# EUROPÄISCHE PATENTANMELDUNG

(11) **EP 3 260 112 A1**
(43) Veröffentlichungstag der Anmeldung: **27.12.2017**
(21) Anmeldenummer: 17000858.5
(22) Anmeldetag: 19.05.2017
(51) Int. Cl.: A61K 8/37, A61Q 19/00, A61Q 3/04, A61K 8/04

(54) **NAGELLACKENTFERNERGEL**

(30) Priorität: 20.05.2016 EP 16170712
(71) Anmelder: Hyga GmbH & Co. KG, 45479 Mülheim (DE)
(72) Erfinder: URBANI, Marcus, 46485 Wesel (DE)
(74) Vertreter: Von Rohr Patentanwälte Partnerschaft mbB

(57) **Zusammenfassung**

Die vorliegende Erfindung betrifft eine Zusammensetzung zur Entfernung von Nagellack in Form eines Gels sowie ein Behältnis zu dessen Applikation.

## Beschreibung

Die vorliegende Erfindung betrifft die technischen Gebiete der Kosmetik sowie der Hygiene, insbesondere der Entfernung von Kosmetikartikeln, wie beispielsweise Nagellacken.

Insbesondere betrifft die vorliegende Erfindung eine Zusammensetzung zur Entfernung von Nagellack in Form eines Gels.

Weiterhin betrifft die vorliegende Erfindung ein Behältnis, welches den gelförmigen Nagellackentferner nach der Erfindung enthält.

Nagellackentferner sind üblicherweise niedrigviskose Flüssigkeiten, welche organische Lösemittel als Hauptbestandteil aufweisen. Sie werden verwendet, um ausgehärtete Nagellacke von Finger- und Fußnägeln zu entfernen, beispielsweise um beschädigten Lack zu erneuern oder eine erneute Lackierung aus ästhetischen Gründen vorzubereiten.

Nagellackentferner sollten einerseits den Nagellack möglichst rasch und vollständig von den Nägeln entfernen und andererseits den Nagel nach Möglichkeit für eine erneute Lackierung vorbereiten. Nagellackentferner werden üblicherweise in kleinen Behältnissen mit Schraubverschluss vertrieben. Die Aufbringung des Nagellackentferners auf den lackierten Finger- bzw. Fußnagel erfolgt entweder durch Applikationsschwämmchen oder mittels getränkter Wattepads. Vereinzelt sind mittlerweile auch mit Nagellackentferner getränkte, einzeln verpackte Tücher oder Wattepads erhältlich.

Den zuvor genannten Applikationsmethoden ist gemein, dass eine optimale Dosierung des Nagellackentferners nur schwer zu erreichen ist und daher üblicherweise zu große Mengen Nagellackentferner verwendet werden. Dies führt einerseits zu einer Verschwendung des Produkts und andererseits oftmals zu einem unerwünschten Kontakt von Nagellackentferner und Haut, was die Haut aufgrund der verwendeten organischen Lösemittel austrocknet und schädigt.

Darüber hinaus führt eine zu hohe Dosierung des Nagellackentferners oftmals auch zu einer Diffusion des angelösten bzw. aufgelösten Nagellackes durch das Wattepad bzw. das Tuch hindurch, so dass die Finger der Anwenderin bzw. des Anwenders mit dem Nagellack gefärbt werden. Diese ungewollte Färbung ist ästhetisch wenig ansprechend und sorgt für Frust beim Anwender.

Die Verwendung dünnflüssiger, niedrigviskoser Nagellackentferner hat darüber hinaus den Nachteil, dass die Substanz versehentlich verschüttet werden kann und partikelförmige Additive, beispielsweise bestimmte Pflegestoffe, welche dem Nagellackentferner zugesetzt werden, sedimentieren.

Weiterhin weisen Aceton- oder Ethylacetat-basierte Nagellackentferner einen oftmals sehr starken Eigengeruch auf, welcher als störend oder künstlich bzw. chemisch wahrgenommen und abgelehnt wird. Aceton und Ethylacetat bzw. Essigsäureethylester finden jedoch aufgrund ihrer guten Eigenschaften als Lösemittel immer noch weitverbreitet Anwendung in Nagellackentfernern.

Die Verwendung dünnflüssiger Nagellacke führt auch dazu, dass mit den gängigen Systemen zur Applikation von Nagellacken eine bequeme einhändige Anwendung durch das Öffnen bzw. Schließen von Drehverschlüssen oder die Entnahme einzeln verpackter Pads oder Tücher nicht möglich ist.

Um zumindest einige der vorgenannten, mit der Applikation des Nagellackentferners verbundenen Nachteile zu überwinden, wurden gelförmige bzw. pastöse Nagellackentferner entwickelt, welche jedoch bislang nicht zur Marktreife gelangen konnten.

So beschreibt die EP 1 591 101 A1 einen gelförmigen oder pastösen Nagellackentferner auf Basis von Ethylacetat und Cellulosederivaten bzw. Harnstoffmethanderivate als Verdickungsmittel bzw. Thixotropiermittel. Die Nagellackentfernerzusammensetzung enthält vorzugsweise 95 bis 97 Gew.-% organische Lösemittel. Nachteilig an den beschriebenen Systemen ist der hohe Anteil an organischen Lösemitteln, welche einen starken Eigengeruch aufweisen sowie Haut und Nägel schädigen, insbesondere austrocknen.

Auch die WO 2015/135021 A1 offenbart einen Nagellackentferner auf Basis von Aceton und/oder Ethylacetat als Lösemittel sowie Hydroxypropylcellulose als Verdickungsmittel. Die Zusammensetzung benötigt eine sehr lange Einwirkzeit, da das Lösemittel nur zeitverzögert aus der Gelmatrix an den Nagel abgegeben wird, weshalb das System für die praktische Anwendung nicht geeignet ist.

Im Stand der Technik fehlt es somit weiterhin an Nagellackentfernern, welche hautverträglich sind, Nagellack schnell und sicher lösen bzw. entfernen sowie einfach in der Anwendung sind. Gleichermaßen sind die in den handelsüblichen Nagellackentfernern verwendeten Lösemittel oftmals aus Gesichtspunkten des Umweltschutzes bedenklich und sollten nicht verwendet werden.

Der vorliegenden Erfindung liegt daher die Aufgabe zugrunde, einen Nagellackentferner, insbesondere ein Nagellackentfernergel, zur Verfügung zu stellen, wobei die zuvor geschilderten, im Zusammenhang mit den im Stand der Technik auftretetenden Probleme und Nachteile zumindest weitgehend vermieden oder wenigstens abgeschwächt werden sollen.

Insbesondere ist eine Aufgabe der vorliegenden Erfindung darin zu sehen, einen Nagellackentferner bereitzustellen, welcher gut dosierbar und gezielt lokal begrenzt anwendbar ist.

Darüber hinaus ist eine Aufgabe der vorliegenden Erfindung darin zu sehen, einen Nagellackentferner bereitzustellen, welcher haut- und nagelverträglich sowie unter Gesichtspunkten des Umweltschutzes unbedenklich ist, gleichwohl Nagellack hervorragend zu lösen vermag.

Eine weitere Aufgabe der vorliegenden Erfindung ist darin zu sehen, einen Nagellackentferner bereitzustellen, welcher Nagellack gründlich und rasch entfernt und darüber hinaus die Nägel nach Möglichkeit optimal auf die weitere Behandlung vorbereitet.

Schließlich ist eine weitere Aufgabe der vorliegenden Erfindung darin zu sehen, ein Behältnis bereitzustellen, welches eine optimale Dosierung und einfache Applikation eines Nagellackes ermöglicht.

Die zuvor geschilderte Aufgabenstellung wird erfindungsgemäß durch eine Zusammensetzung nach Anspruch 1 gelöst; weitere vorteilhafte Weiterbildungen und Ausgestaltungen der erfindungsgemäßen Zusammensetzung sind Gegenstand der diesbezüglichen Unteransprüche.

Weiterer Gegenstand der vorliegenden Erfindung ist ein Behältnis nach Anspruch 17.

Es versteht sich von selbst, dass Besonderheiten, Merkmale, Ausgestaltungen und Ausführungsformen sowie Vorteile oder dergleichen, welche nachfolgend - zu Zwecken der Vermeidung von unnötigen Wiederholungen - nur zu einem Erfindungsaspekt ausgeführt werden, selbstverständlich in Bezug auf die übrigen Erfindungsaspekte entsprechend gelten, ohne dass es einer ausdrücklichen Erwähnung bedarf.

Weiterhin versteht es sich von selbst, dass bei der nachfolgenden Angabe von Werten, Zahlen und Bereichen die diesbezüglichen Werte-, Zahlen- und Bereichsangaben nicht beschränkt zu verstehen sind; es versteht sich für den Fachmann vielmehr von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder mit dem Fachmann auf diesem Gebiet an sich geläufigen Bestimmungsmethoden ermittelt bzw. bestimmt werden können.

Darüber hinaus versteht es sich von selbst, dass alle gewichts- oder mengenbezogenen Prozentangaben vom Fachmann derart ausgewählt werden, dass in der Summe 100 % resultieren, dies versteht sich jedoch von selbst.

Dies vorausgeschickt, wird im Folgenden die vorliegende Erfindung näher beschrieben.

Gegenstand der vorliegenden Erfindung ist somit - gemäß einem **ersten** Aspekt der vorliegenden Erfindung - eine Zusammensetzung zur Entfernung von Nagellack in Form eines Gels, wobei die Zusammensetzung wässrig basiert ist und mindestens einen Ester einer C₃- bis C₅-Carbonsäure enthält.

Die Verwendung eines gelförmigen Nagellackentferners ermöglicht eine genaue und gezielte Dosierung der eingesetzten Menge an Nagellackentferner. Auf diese Weise kann ein "Durchfärben" des gelösten Nagellackes auf die Finger des Anwenders vermieden werden.

Durch die hohe Viskosität des erfindungsgemäßen Nagellackentferners kann auch der exzessive Kontakt von Nägeln und insbesondere auch der Haut mit dem Nagellackentferner vermieden werden, so dass insbesondere ein Austrocknen der Haut weitgehend vermieden werden kann.

Insbesondere bei Verwendung thixotroper Zusammensetzungen, welche unter Energieeintrag in einen niedrigviskosen Zustand übergehen, ist eine einfache einhändige Applikation und Dosierung, beispielsweise über übliche Pumpspender, der Zusammensetzung möglich. Durch den Energieeintrag im Pumpspender während des Pumpvorgangs geht das Gel in einen niedrigviskosen Zustand über und kann beispielsweise einfach mit einem Wattepad aufgenommen werden, auf welchem es wieder in den hochviskosen Zustand übergeht. So wird das Wattepad zwar oberflächlich von dem Nagellackentferner benetzt, jedoch nicht vollständig durchtränkt, so dass die Hände des Nutzers auch ohne Schutzmaßnahmen, wie beispielsweise die Verwendung von Einmalhandschuhen, frei von Lösemitteln bleiben. Auf diese Weise kann auch das ästhetisch wenig ansprechende Färben der Finger des Nutzers durch Berührung des Wattepads vermieden werden. Gleichfalls können kleine wiederverschließbare Behältnisse, insbesondere Tuben, mit dem Nagellackentfernergel gefüllt werden, welche insbesondere die Applikation unterwegs bzw. auf Reisen ermöglichen, wenn vorzugsweise nur eine kleine Menge an Nagellackentfernerpad mitgeführt werden soll.

Darüber hinaus wird auch eine ungewollte Verteilung des ungelösten Nagellacks auf den Fingern des Anwenders durch die Verwendung eines thixotropen bzw. gelförmigen Nagellackentferners vermieden.

Die Verwendung einer gelförmigen Zusammensetzung erlaubt darüber hinaus die gleichmäßige und lagerstabile Verteilung von größeren Partikeln, insbesondere von sogenannten Pflegeperlen, welche pflegende Substanzen in einer Membranhülle enthalten, oder von Glanzpigmenten. Auch über längere Lagerzeiträume bleiben die Additive in der Zusammensetzung gleichmäßig und homogen dispergiert, ohne dass Sedimentation eintritt.

Durch die gleichmäßige Verteilung von pflegenden Substanzen, können die Nägel nicht nur von Nagellack befreit und gereinigt, sondern gleichzeitig desinfiziert, gepflegt und gegebenenfalls gehärtet werden. Auch kann die umgebende Haut direkt gepflegt werden. Es können somit unterschiedliche Arbeitsschritte bei der Nagelpflege durch Verwendung eines Gels zusammengefasst und vereinheitlicht werden.

Durch die Verwendung von Estern der C₃- bis C₅-Carbonsäure kann insbesondere auf Aceton und Ethylacetat als Lösemittel verzichtet werden. Aceton und Ethylacetat bzw. Essigsäureethylester schädigen die Haut, besitzen einen unangenehmen penetranten Eigengeruch und sollten unter Gesichtspunkten des Umweltschutzes nicht verwendet werden. Die im Rahmen der vorliegenden Erfindung verwendeten Ester von Carbonsäuren als Lösemittel für Nagellacke sind ungiftig, unter Gesichtspunkten des Umweltschutzes unbedenklich und insbesondere bei Verwendung von Milchsäureestern auf Basis nachwachsender Rohstoffe ausgebildet.

Darüber hinaus zeichnet sich die erfindungsgemäße Zusammensetzung durch ein hohes Lösevermögen für Nagellacke aus. Insbesondere wird eine 0,5 mm dicke Nagellackschicht in Fingernagelgröße nach einer Einwirkzeit der erfindungsgemäßen Zusammensetzung von weniger als 10 Sekunden, vollständig entfernt. Dies entspricht einem normalen Nagellackentfernungsvorgang und wird vom Verbraucher nicht als störend oder verzögert wahrgenommen. Die erfindungsgemäße Zusammensetzung ermöglicht somit eine einfache, rasche und vollständige Entfernung von Nagellacken.

Durch die gelartige Textur wird darüber hinaus der Eigengeruch der verwendeten Lösemittel deutlich verringert und ein versehentliches Verschütten der Zusammensetzung ist nahezu ausgeschlossen. Die erfindungsgemäße Zusammensetzung kann somit im Vergleich zu herkömmlichen Nagellackentfernern deutlich sicherer verwendet werden.

Unter einem "Gel" sollen im Rahmen der vorliegenden Erfindung formbeständige, leicht deformierbare an Flüssigkeit reiche disperse Systeme aus mindestens zwei Komponenten verstanden werden. Insbesondere bestehen Gele üblicherweise aus zumindest einem festen, kolloidal verteilten Stoff mit langen oder stark verzweigten Teilchen und einer Flüssigkeit, insbesondere Wasser oder einem organischen Lösemittel, als Dispersionsmittel. Die feste Substanz ist üblicherweise kohärent, d. h. sie bildet im Dispersionsmittel ein räumliches Netzwerk, wobei die Teilchen durch Neben- oder Hauptvalenzen an verschiedenen Punkten, den sogenannten Haftpunkten, aneinanderhaften. Für weitergehende Einzelheiten zu dem Begriff "Gel" kann bewiesen werden auf Römpp, Chemielexikon, Band 2, 10. Auflage, Georg Thieme Verlag, Stuttgart/New York, 1990, Georg Thieme Verlag, Seite 1311 unter dem Stichwort "Gele".

Im Rahmen der vorliegenden Erfindung hat es sich bewährt, wenn die Zusammensetzung thixotrop ist. Unter dem Begriff "thixotrop" ist dabei im Rahmen der vorliegenden Erfindung zu verstehen, dass das Gel unter Einwirkung mechanischer Kräfte, zum Beispiel beim Rühren, Schütteln, unter Ultraschalleinwirkung oder beim Pumpen durch einen Pumpspender, verflüssigt wird und sich nach Beendigung der mechanischen Beanspruchung wieder verfestigt. Die Viskosität des Gels nimmt insbesondere unter dem Einfluss zunehmender Schubspannung ab. Für weitergehende Einzelheiten zu dem Begriff "thixotrop" kann verwiesen werden auf Römpp, Chemielexikon, Band 6, 10. Auflage, Georg Thieme Verlag, Stuttgart/New York, 1992, Seite 4597 zum Stichwort "Thixotropie" verwiesen werden.

Die Verwendung thixotroper Gele ermöglicht es, die Zusammensetzung beispielsweise durch Pumpspender sparsam und genau zu dosieren, wobei das durch mechanische Belastung niedrigviskose Gel nach Austritt aus dem Pumpspender wieder eine höhere Viskosität annimmt und ein Wattepad oder Tuch beispielsweise nur benetzt und nicht vollständig durchtränkt. Auch ermöglicht ein thixotropes Gel eine homogene Dispergierung von größeren Partikeln, insbesondere von sogenannten Pflegeperlen, ohne dass diese sedimentieren.

Im Rahmen der vorliegenden Erfindung weist die Zusammensetzung üblicherweise mindestens einen Gelbildner auf. Gelbildner werden synonym auch als Geliermittel, Verdickungsmittel, Viskositätsregulatoren, Reologiemodifizierer oder Thixotrophiermittel bezeichnet und dienen der Einstellung eines gewünschten Viskositätsverhaltens. Bei den Gelbildnern handelt es sich insbesondere um zumeist organische, hochmolekulare Stoffe, welche Flüssigkeiten aufsaugen, dabei aufquellen und schließlich in zähflüssige echte oder kolloide Lösungen übergehen, wobei Gele und Schleime gebildet werden. Für weitere Einzelheiten zu dem Begriff "Gelbildner" kann verwiesen werden auf Römpp, Chemielexikon, Band 6, 10. Auflage, Georg Thieme Verlag, Stuttgart/New York, 1992, Seite 4890 zum Stichwort "Verdickungsmittel".

Insbesondere handelt es sich bei dem Gelbildner erfindungsgemäß um mindestens ein Polymer. Im Rahmen der vorliegenden Erfindung kann es auch vorgesehen sein, dass eine Mischung von mehreren Gelbildnern eingesetzt wird.

Üblicherweise ist der Gelbildner ausgewählt aus der Gruppe von Polyacrylaten, Celluloseethern, Polyacrylamiden, Polyethern oder Polyurethanen.

In diesem Zusammenhang werden besonders gute Ergebnisse erhalten, wenn der Gelbildner ausgewählt ist aus Polyacrylaten. Insbesondere bei Verwendung homopolymerer Acrylsäure als Gelbildner, welche beispielsweise unter der Bezeichnung Carbomer bekannt ist, werden besonders geeignete Gele mit hervorragenden thixotropen Eigenschaften erhalten.

Was nun das Molekulargewicht des verwendeten Gelbildners anbelangt, so kann dieses naturgemäß in weiten Bereichen variieren. Es hat sich jedoch bewährt, wenn der Gelbildner ein gewichtsmittleres Molekulargewicht M_{w} im Bereich von 50.000 bis 5.000.000 g/mol, insbesondere 100.000 bis 3.000.000 g/mol, vorzugsweise 500.000 bis 2.500.000 g/mol, bevorzugt 750.000 bis 2.000.000 g/mol, besonders bevorzugt 1.000.000 bis 1.500.000 g/mol, aufweist. Gelbildner mit Molekulargewichten in den zuvor genannten Bereichen erzielen schon bei äußerst geringem mengenmäßigen Einsatz einen sehr hohen Viskositätseinstieg und sehr gute Thixtropieeigenschaften der Zusammensetzung.

Die Molekulargewichte können im Rahmen der vorliegenden Erfindung insbesondere mittels Gelpermeationschromatographie (GPC) bestimmt werden.

Wenn die Zusammensetzung einen Gelbildner enthält, so enthält die Zusammensetzung den Gelbildner üblicherweise in Mengen von 0,01 bis 10 Gew.-%, insbesondere 0,05 bis 5 Gew.-%, vorzugsweise 0,1 bis 2 Gew.-%, bevorzugt 0,2 bis 1 Gew.-%, besonders bevorzugt 0,3 bis 0,7 Gew.-%, bezogen auf die Zusammensetzung. Insbesondere bei Verwendung der zuvorgenannten hochmolekularen Gelbildner können die Gelbildner in der erfindungsgemäßen Zusammensetzung in äußerst geringen Mengen vorhanden sein und trotzdem die Viskosität der Zusammensetzung in der beabsichtigten Weise bestimmen.

Was nun die Viskosität der erfindungsgemäßen Zusammensetzung anbelangt, so kann diese gleichermaßen in Abhängigkeit von den jeweiligen Anwendungserfordernissen in weiten Bereichen variieren. Es hat sich jedoch gezeigt, dass besonders gute Ergebnisse erhalten werden, wenn die Zusammensetzung eine dynamische Viskosität bei 23 °C und einer Scherrate von 100 s⁻¹ von weniger als 100.000 mPas, insbesondere weniger als 80.000 mPas, vorzugsweise weniger als 70.000 mPas, bevorzugt weniger als 50.000 mPas, aufweist.

Insbesondere werden besonders gute Ergebnisse erhalten, wenn die Zusammensetzung eine dynamische Viskosität bei 20 °C und einer Scherrate von 100 s⁻¹ im Bereich von 10.000 bis 100.000 mPas, insbesondere von 20.000 mPas bis 80.000 mPas, vorzugsweise von 30.000 bis 70.000 mPas, bevorzugt 40.000 bis 50.000 mPas, aufweist.

Die im Rahmen der vorliegenden Erfindung vorstehend angegebenen Viskositäten können insbesondere nach der Methode gemäß EN ISO 2555:2017-05 bestimmt werden.

Was nun den pH-Wert der erfindungsgemäßen Zusammensetzung anbelangt, so kann dieser gleichfalls in weiten Bereichen variieren. Es hat sich jedoch gezeigt, dass besonders leistungsfähige und hautverträgliche Zusammensetzungen erhalten werden, wenn die Zusammensetzung einen pH-Wert im Bereich von 4 bis 8, insbesondere 5 bis 7, aufweist.

Gemäß einer bevorzugten Ausführungsform ist die Zusammensetzung insbesondere zumindest im Wesentlichen frei von Aceton und/oder Essigsäureethylester.

Darüber hinaus ist es gemäß einer weiteren bevorzugten Ausführungsform vorgesehen, dass die Zusammensetzung insbesondere zumindest im Wesentlichen frei ist von Ethanol und/oder Essigsäurebutylester.

Gemäß einer besonders bevorzugten Ausführungsform der vorliegenden Erfindung ist die Zusammensetzung insbesondere zumindest im Wesentlichen frei von Aceton, Ethanol, Essigsäureethylester und/oder Essigsäurebutylester.

Wie zuvor bereits ausgeführt, enthält die Zusammensetzung mindestens einen Ester einer C₃- bis C₅-Carbonsäure. Besonders gute Ergebnisse werden erhalten, wenn die Zusammensetzung einen Ester einer C₃- bis C₅-Carbonsäure mit C₁- bis C₈-Alkoholen, insbesondere C₁- bis C₆-Alkoholen, vorzugsweise C₂- bis C₄-Alkoholen, bevorzugt C₂- und/oder C₄-Alkoholen, enthält. Die im Rahmen der vorliegenden Erfindung für die Veresterung verwendeten Alkohole können dabei linear oder verzweigt sein. Vorzugsweise handelt es sich bei den Alkoholen um Alkanole.

Im Rahmen der vorliegenden Erfindung hat es sich insbesondere bewährt, wenn die Zusammensetzung einen Ester der Milchsäure mit C₁- bis C₈-Alkoholen, insbesondere mit C₁- bis C₆-Alkoholen, vorzugsweise C₂- bis C₄-Alkoholen, bevorzugt C₂- und/oder C₄-Alkoholen, enthält. Insbesondere Lactatester zeigen einerseits eine hohe Lösekraft für Nagellacke und sind andererseits haut- und umweltverträglich.

Gemäß einer bevorzugten Ausführungsform der vorliegenden Erfindung enthält die Zusammensetzung Ethyllactat und/oder Butyllactat. Besonders bevorzugt ist es in diesem Zusammenhang, wenn die Zusammensetzung sowohl Ethyllactat als auch Butyllactat enthält.

Wenn die Zusammensetzung Ethyllactat und Butyllactat enthält, so hat es sich bewährt, wenn die Zusammensetzung Ethyllactat und Butyllactat in einem gewichtsbezogenen Verhältnis von Ethyllactat zu Butyllactat im Bereich von 1 : 1 bis 100 : 1, insbesondere 5 : 1 bis 70 : 1, vorzugsweise 10: 1 bis 50 : 1, bevorzugt 15 : 1 bis 40 : 1, besonders bevorzugt 20 : 1 bis 35 : 1, ganz besonders bevorzugt 25 : 1 bis 30: 1, enthält. Insbesondere in den vorgenannten mengenmäßigen Verhältnissen werden sowohl hervorragende Löseeigenschaften für Nagellacke als auch eine gute dermatologische Verträglichkeit und optimale rheologische Eigenschaften der Zusammensetzung erhalten.

Was nun die Menge an Ester einer C₃- bis C₅-Carbonsäure in der erfindungsgemäßen Zusammensetzung anbelangt, so kann diese gleichfalls in Abhängigkeit von den jeweiligen Erfordernissen in weiten Bereichen variieren. Es hat sich jedoch gezeigt, dass besonders gute Ergebnisse erhalten werden, wenn die Zusammensetzung den Ester einer C₃- bis C₅-Carbonsäure in Mengen von 40 bis 95 Gew.-%, insbesondere 50 bis 85 Gew.-%, vorzugsweise 60 bis 80 Gew.-%, bevorzugt 60 bis 75 Gew.-%, bezogen auf die Zusammensetzung, enthält. Die erfindungsgemäße Zusammensetzung weist somit vorzugsweise relativ geringe Mengen an organischem Lösemittel auf, welches darüber hinaus bevorzugt vollständig aus nachwachsenden Rohstoffen erhältlich und biologisch abbaubar ist.

Gemäß einer besonderen Ausführungsform der vorliegenden Erfindung kann die Zusammensetzung mindestens ein weiteres organisches Lösemittel aufweisen. Falls die Zusammensetzung ein weiteres organisches Lösemittel aufweist, so wird üblicherweise ein Teil des Esters einer C₃- bis C₅-Carbonsäure durch das organische Lösemittel ersetzt, d. h. die Gesamtmenge an Ester einer C₃- bis C₅-Carbonsäure sowie des weiteren organischen Lösemittels entspricht gemäß dieser Ausführungsform den zuvor genannten Mengen an Ester einer C₃- bis C₅-Carbonsäure.

Falls die Zusammensetzung ein weiteres organisches Lösemittel enthält, so weist die Zusammensetzung das weitere organische Lösemittel üblicherweise in Mengen von 1 bis 30 Gew.-%, insbesondere 2 bis 25 Gew.-%, vorzugsweise 5 bis 20 Gew.-%, bevorzugt 5 bis 10 Gew.-%, bezogen auf die Zusammensetzung, auf.

Falls die Zusammensetzung ein weiteres organisches Lösemittel aufweist, so ist dieses üblicherweise ausgewählt aus Glycolen, insbesondere Propylenglycol, und/oder Isopropanol. Glycole und Isopropanol weisen im Vergleich zu vielen anderen organischen Lösemitteln gute Löseeigenschaften bei gleichzeitiger guter dermatologischer Verträglichkeit auf.

Wie zuvor ausgeführt, enthält die erfindungsgemäße Zusammensetzung Wasser. Üblicherweise weist die Zusammensetzung Wasser in Mengen von 1 bis 60 Gew.-%, vorzugsweise 2 bis 50 Gew.-%, bevorzugt 5 bis 40 Gew.-%, besonders bevorzugt 15 bis 35 Gew.-%, ganz besonders bevorzugt 20 bis 30 Gew.-%, bezogen auf die Zusammensetzung, auf. Die erfindungsgemäße Zusammensetzung erhält somit einen relativ hohen Anteil an Wasser, welcher sich insbesondere positiv auf die Ausbildung der Gelstruktur, speziell auf das Quellverhalten der eingesetzten Gelbildner, auswirkt. Darüber hinaus ist Wasser physiologisch und unter Gesichtspunkten des Umweltschutzes unbedenklich.

Gemäß einer bevorzugten Ausführungsform der vorliegenden Erfindung weist die Zusammensetzung mindestens eine pflegende Substanz, insbesondere zur Haut- und/oder Nagelpflege, auf.

Wenn die erfindungsgemäße Zusammensetzung eine pflegende Substanz aufweist, so kann es vorteilhafterweise vorgesehen sein, dass die pflegende Substanz in Mikrokapseln, sogenannten Pflegeperlen, enthalten ist. Dabei wird die pflegende Substanz in einer Schicht bzw. Membran aus Chitosan, Polylactid, Cellulosederivaten oder Alginat eingeschlossen, gegebenenfalls in einer 01-in-Wasser-Emulsion oder Wasser-in-Öl-Emulsion. Die Kapseln bzw. Pflegeperlen werden bei der Anwendung durch mechanische Belastung zerstört und geben ihre Inhaltsstoffe frei. Auf diese Weise ist es möglich, die zum Teil sehr empfindlichen pflegenden Substanzen vor Oxidation bzw. dem Einfluss des Lösemittels zu schützen.

Was nun die pflegenden Substanzen anbelangt, so können diese aus den im Bereich der Kosmetik oder Dermatologie üblichen pflegenden Substanzen ausgewählt sein. Insbesondere können die pflegenden Substanzen ausgewählt sein aus Vitaminen, Ölen und pflanzlichen Extrakten, Hyaluronsäure, Panthenol und Mineralien.

Besonders gute Ergebnisse werden erhalten, wenn die pflegende Substanz ausgewählt ist aus Feuchtigkeitsspendern, auch Emollienten genannt, und/oder Antioxidantien. Die Feuchtigkeitsspender können dabei insbesondere ausgewählt sein aus natürlichen und synthetischen Feuchtigkeitsspendern. Synthetische Feuchtigkeitsspender sind beispielsweise Phthalate, wie Dibutylphthalat oder Dioctylphtalat, aber auch Butylstearat oder Dibutyltatrat. Natürliche Feuchtigkeitsspender sind beispielsweise Hyaluronsäure oder Panthenol. Im Rahmen der vorliegenden Erfindung wird es bevorzugt, wenn der Feuchtigkeitsspender ein natürlicher Feuchtigkeitsspender ist, insbesondere ausgewählt aus Hyaluronsäure und/oder Panthenol oder deren pharmakologisch und/oder dermatologisch unbedenklichen Derivaten und Salzen.

Darüber hinaus hat es sich bewährt, wenn das Antioxidans ausgewählt ist aus Vitaminen, Allontoin, Betacarotin, Lutein, Lycopin. Besonders bevorzugt ist es, wenn das Antioxidans ausgewählt ist aus Vitaminen oder deren pharmakologisch und/oder dermatologisch unbedenklichen Derivaten und Salzen. Insbesondere ist das Antioxidans ausgewählt aus Ascorbinsäure und/oder Tocopherol. Bevorzugt handelt es sich bei dem Antioxidans um Ascorbylstearat und/oder Tocopherolacetat, bevorzugt Tocopherolacetat.

Wenn die Zusammensetzung eine pflegende Substanz enthält, so weist die Zusammensetzung die pflegende Substanz im Allgemeinen in Mengen von 0,01 bis 5 Gew.-%, insbesondere 0,05 bis 2 Gew.-%, vorzugsweise 0,1 bis 1 Gew.-%, bevorzugt 0,15 bis 5 Gew.-%, bezogen auf die Zusammensetzung, auf.

Darüber hinaus kann es vorgesehen sein, dass die Zusammensetzung mindestens ein desinfizierendes Mittel aufweist. Insbesondere kann es vorgesehen sein, dass die Zusammensetzung ein Desinfektionsmittel enthält. Das desinfizierende Mittel kann im Rahmen der vorliegenden Erfindung eine Einzelsubstanz oder ein Gemisch verschiedener Substanzen sein.

Wenn die Zusammensetzung ein desinfizierendes Mittel enthält, so hat es sich als zweckmäßig erwiesen, wenn die Zusammensetzung das desinfizierende Mittel in Mengen von 0,0001 bis 1 Gew.-%, insbesondere 0,001 bis 0,5 Gew.-%, vorzugweise 0,003 bis 0,1 Gew.-%, bevorzugt 0,007 bis 0,05 Gew.-%, bezogen auf die Zusammensetzung, aufweist.

Wenn die Zusammensetzung ein desinfizierendes Mittel aufweist, so ist dieser üblicherweise ausgewählt aus Chlorhexidin, Octenidin und pflanzlichen Ölen, insbesondere pflanzlichen Ölen. Insbesondere kann es vorgesehen sein, dass ätherische Öle, insbesondere der Edeltanne, des Eukalyptus, des Lorbeer, der Myrrhe, des Thymian, der Myrte, oder Orangenöl, Kiefernnadelöl oder Teebaumöl als desinfizierende Mittel in geringen Mengen der erfindungsgemäßen Zusammensetzung beigemischt werden.

Darüber hinaus kann es vorgesehen sein, dass die Zusammensetzung mindestens ein Additiv, insbesondere in Mengen von 0,01 bis 10 Gew.-%, vorzugsweise 0,1 bis 8 Gew.-%, bevorzugt 0,5 bis 5 Gew.-%, besonders bevorzugt 1 bis 3 Gew.-%, bezogen auf die Zusammensetzung, enthält.

Üblicherweise ist das Additiv ausgewählt aus pH-Regulatoren, Konservierungsmitteln, Geruchs- und/oder Farbstoffen, insbesondere pH-Regulatoren, Geruchs- und/oder Farbstoffen.

Gegenstand der vorliegenden Erfindung ist somit eine zuvor beschriebene Zusammensetzung, wobei die Zusammensetzung
(a) mindestens einen Gelbildner in Mengen von 0,01 bis 10 Gew.-%, bezogen auf die Zusammensetzung,
(b) mindestens einen Ester einer C₃- bis C₅-Carbonsäure in Mengen von 40 bis 95 Gew.-%, bezogen auf die Zusammensetzung,
(c) Wasser in Mengen von 1 bis 60 Gew.-%, bezogen auf die Zusammensetzung,
(d) mindestens eine pflegende Substanz in Mengen von 0,01 bis 5 Gew.-%, bezogen auf die Zusammensetzung,
(e) mindestens ein desinfizierendes Mittel in Mengen von 0,0001 bis 1 Gew.-%, bezogen auf die Zusammensetzung, und
(f) mindestens ein Additiv in Mengen von 0,01 bis 10 Gew.-%, bezogen auf die Zusammensetzung,
enthält.

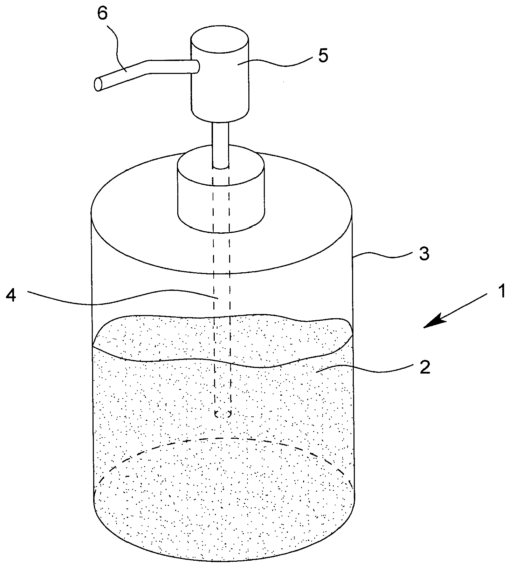

Es zeigt, die einzige Figurendarstellung gemäß
- Fig. 1: eine Applikationsform der erfindungsgemäßen Zusammensetzung, welche besonders gut dosierbar ist.

Weiterer Gegenstand der vorliegenden Erfindung - gemäß einem **zweiten** Aspekt der vorliegenden Erfindung - ist ein Behältnis, insbesondere ein Pumpspender oder eine Tube, aufweisend die zuvor beschriebene Zusammensetzung.

Für weitere Einzelheiten zu dem erfindungsgemäßen Behältnis kann auf die vorherigen Ausführungen zu der erfindungsgemäßen Zusammensetzung verwiesen werden, welche in Bezug auf das erfindungsgemäße Behältnis entsprechend gelten.

Die vorliegende Erfindung wird nachfolgend anhand einer bevorzugten Ausführungsform gemäß der Figurenbeschreibung weiter verdeutlicht, ohne den Gegenstand der vorliegenden Erfindung jedoch auf die Figurenbeschreibung zu beschränken.

Es zeigt die einzige Figurendarstellung gemäß Figur 1 eine besonders bevorzugte Applikationsform, insbesondere einen Pumpspender 1, welcher die erfindungsgemäße Zusammensetzung 2 in einem Behältnis 3 enthält. Das Behältnis 3 hat üblicherweise ein Volumen von 15 bis 250 ml, insbesondere 25 bis 200 ml, vorzugsweise 50 bis 100 ml. Dies entspricht einer gut zu handhabenden und in einem angemessenen Zeitraum zu verbrauchenden Menge der erfindungsgemäßen Zusammensetzung.

Die Zusammensetzung 2 ist ein hochviskoses thixotropes Nagellackentfernergel, welches Partikel in Form von Pflegeperlen enthält. Die Pflegeperlen enthalten Panthenol und Tecopherolacetat in einer Chitosankapsel.

In die Zusammensetzung 2 taucht ein Steigrohr 4 ein, welches mit einer Hubkolbenpumpe 5, wie sie in handelsüblichen Seifenspendern eingesetzt wird, verbunden ist. An die Hubkolbenpumpe 5 schließt sich ein Auslass 6 an, durch welchen das Gel abgegeben werden kann.

Durch betätigen der Hubkolbenpumpe 5, welche nicht im Detail in der Figurendarstellung dargestellt ist, wird eine mechanische Belastung auf die Zusammensetzung 2 ausgeübt, welche sich aufgrund ihrer thixotropen Eigenschaften verflüssigt und über den Auslass 6 an die Umgebung, insbesondere ein Wattepad, abgegeben werden kann.

Aufgrund der thixotropen Eigenschaften verfestigt sich die Zusammensetzung 2 nach Beendigung der mechanischen Beanspruchung, so dass das Wattepad nicht vollständig von der Zusammensetzung 2 durchtränkt wird, sondern diese nur oberflächlich benetzt.

Der Pumpspender 1 kann bequem mit einer Hand bedient werden und ist gegen ein Auslaufen des Gels gesichert, da dieses hochviskos ist. Ein unbeabsichtigtes Verschütten des Nagellackentferners kann somit nicht erfolgen.

Gemäß einer weiteren bevorzugten, jedoch nicht in der Figurendarstellung dargestellten Ausführungsform der vorliegenden Erfindung ist das Behältnis eine Tube, insbesondere eine verschließbare Tube. In diesem Zusammenhang kann es vorgesehen sein, dass die Tube mit einem Schraubverschluss ausgestattet ist.

Falls das Behältnis in Form einer Tube ausgebildet ist, ist es vorzugsweise vorgesehen, dass das Behältnis ein Volumen von 10 bis 200 ml, insbesondere 20 bis 50 ml, vorzugsweise 25 bis 40 ml, aufweist. Derartige Tuben eignen sich insbesondere für die Anwendung unterwegs bzw. auf Reisen, bei welchen lediglich eine geringe Menge an Nagellackentfernergel mitgeführt werden soll. Durch die Thixotropie des Nagellackentfernergels wird verhindert, dass das Nagellackentfernergel nach Entfernung nach Öffnen der Tube, insbesondere nach Öffnung des Schraubverschlusses, unkontrolliert aus dem Behältnis herausfließt, sondern genau dosiert abgegeben werden kann.

Der Gegenstand der vorliegenden Erfindung wird nachfolgend weiter in nicht beschränkender Weise durch die Ausführungsbeispiele verdeutlicht.

### AUSFÜHRUNGSBEISPIELE:

Der Gegenstand der vorliegenden Erfindung wird nachfolgend an Beispielrezeptoren verdeutlicht.

**Beispielrezeptur 1:**

| **Inhaltsstoff** | **Gew.-%** | **Eigenschaft** |
|---|---|---|
| Carbomer | 0,5 | Gelbildner |
| Ethyllactat | 67 | Lösemittel |
| Butyllactat | 2 | Lösemittel |
| Tocopherolacetat | 0,2 | Antioxidans |
| pH-Regulator | 0,3 | pH-Regulator |
| Teebaumöl | 0,01 | Desinfektionsmittel |
| Duft- und Farbstoffe | 3 | Duft- und Farbstoffe |
| Wasser | ad 100 | Lösemittel |

**Beispielrezeptur 2:**

| **Inhaltsstoff** | **Gew.-%** | **Eigenschaft** |
|---|---|---|
| Carbomer | 0,5 | Gelbildner |
| Ethyllactat | 67 | Lösemittel |
| Butyllactat | 2 | Lösemittel |
| Panthenol | 0,2 | Feuchtigkeitsspender |
| pH-Regulator | 0,3 | pH-Regulator |
| Teebaumöl | 0,01 | Desinfektionsmittel |
| Duft- und Farbstoffe | 3 | Duft- und Farbstoffe |
| Wasser | ad 100 | Lösemittel |

Die Zusammensetzungen gemäß den Beispielrezepturen weisen dynamische Viskositäten bei 20 °C von 45.000 mPas auf. Die Viskositäten werden auf einem Fungillab-Rotationsviskosimeter "Viscolead" mit der Spindel Nr. 6 bestimmt.

**Bezugszeichenliste:**

| | | | |
|---|---|---|---|
| 1 | Pumpspender | 4 | Steigrohr |
| 2 | Zusammensetzung | 5 | Hubkolbenpumpe |
| 3 | Behältnis | 6 | Auslass |

## Patentansprüche

1. Zusammensetzung zur Entfernung von Nagellack in Form eines Gels
**dadurch gekennzeichnet,**
**dass** die Zusammensetzung wässrig basiert ist und mindestens einen Ester einer C₃- bis C₅-Carbonsäure enthält.

2. Zusammensetzung nach Anspruch 1, **dadurch gekennzeichnet, dass** die Zusammensetzung thixotrop ist.

3. Zusammensetzung nach Anspruch 1 oder 2, **dadurch gekennzeichnet, dass** die Zusammensetzung mindestens einen Gelbildner aufweist.

4. Zusammensetzung nach Anspruch 3, **dadurch gekennzeichnet, dass** die Zusammensetzung den Gelbildner in Mengen von 0,01 bis 10 Gew.-%, insbesondere 0,05 bis 5 Gew.-%, vorzugsweise 0,1 bis 2 Gew.-%, bevorzugt 0,2 bis 1 Gew.-%, besonders bevorzugt 0,3 bis 0,7 Gew.-%, bezogen auf die Zusammensetzung, aufweist.

5. Zusammensetzung nach einem der vorangehenden Ansprüche, **dadurch gekennzeichnet, dass** die Zusammensetzung einen pH-Wert im Bereich von 4 bis 8, insbesondere 5 bis 7, aufweist.

6. Zusammensetzung nach einem der vorangehenden Ansprüche, **dadurch gekennzeichnet, dass** die Zusammensetzung insbesondere zumindest im Wesentlichen frei ist von Aceton und/oder Essigsäureethylester.

7. Zusammensetzung nach einem der vorangehenden Ansprüche, **dadurch gekennzeichnet, dass** die Zusammensetzung insbesondere zumindest im Wesentlichen frei ist von Ethanol und/oder Essigsäurebutylester.

8. Zusammensetzung nach einem der vorangehenden Ansprüche, **dadurch gekennzeichnet, dass** die Zusammensetzung einen Ester einer C₃- bis C₅-Carbonsäure mit C₁- bis C₈-Alkoholen, insbesondere C₁- bis C₆-Alkoholen, vorzugsweise C₂- bis C₄-Alkoholen, bevorzugt C₂- und/oder C₄-Alkoholen, enthält.

9. Zusammensetzung nach einem der vorangehenden Ansprüche, **dadurch gekennzeichnet, dass** die Zusammensetzung den Ester einer C₃- bis C₅-Carbonsäure in Mengen von 40 bis 95 Gew.-%, insbesondere 50 bis 85 Gew.-%, vorzugsweise 60 bis 80 Gew.-%, bevorzugt 65 bis 75 Gew.-%, bezogen auf die Zusammensetzung, enthält.

10. Zusammensetzung nach einem der vorangehenden Ansprüche, **dadurch gekennzeichnet, dass** die Zusammensetzung mindestens ein organisches Lösemittel aufweist.

11. Zusammensetzung nach einem der vorangehenden Ansprüche, **dadurch gekennzeichnet, dass** die Zusammensetzung Wasser in Mengen von 1 bis 60 Gew.-%, vorzugsweise 2 bis 50 Gew.-%, bevorzugt 5 bis 40 Gew.-%, besonders bevorzugt 15 bis 35 Gew.-%, ganz besonders bevorzugt 20 bis 30 Gew.-%, bezogen auf die Zusammensetzung, enthält.

12. Zusammensetzung nach einem der vorangehenden Ansprüche, **dadurch gekennzeichnet, dass** die Zusammensetzung mindestens eine pflegende Substanz, insbesondere zur Haut- und/oder Nagelpflege, aufweist.

13. Zusammensetzung nach einem der Ansprüche 23 bis 26, **dadurch gekennzeichnet, dass** die Zusammensetzung die pflegende Substanz in Mengen von 0,01 bis 5 Gew.-%, insbesondere 0,05 bis 2 Gew.-%, vorzugsweise 0,1 bis 1 Gew.-%, bevorzugt 0,15 bis 0,5 Gew.-%, bezogen auf die Zusammensetzung, aufweist.

14. Zusammensetzung nach einem der vorangehenden Ansprüche, **dadurch gekennzeichnet, dass** die Zusammensetzung mindestens ein desinfizierendes Mittel aufweist.

15. Zusammensetzung nach einem der vorangehenden Ansprüche, **dadurch gekennzeichnet, dass** die Zusammensetzung mindestens ein Additiv, insbesondere in Mengen von 0,01 bis 10 Gew.-%, vorzugsweise 0,1 bis 8 Gew.-%, bevorzugt 0,5 bis 5 Gew.-%, besonders bevorzugt 1 bis 3 Gew.-%, bezogen auf die Zusammensetzung, enthält.

16. Zusammensetzung nach einem der vorangehenden Ansprüche, **dadurch gekennzeichnet, dass** die Zusammensetzung
(a) mindestens einen Gelbildner in Mengen von 0,01 bis 10 Gew.-%, bezogen auf die Zusammensetzung,
(b) mindestens einen Ester einer C₃- bis C₅-Carbonsäure in Mengen von 40 bis 95 Gew.-%, bezogen auf die Zusammensetzung,
(c) Wasser in Mengen von 1 bis 60 Gew.-%, bezogen auf die Zusammensetzung,
(d) mindestens eine pflegende Substanz in Mengen von 0,01 bis 5 Gew.-%, bezogen auf die Zusammensetzung,
(e) mindestens ein desinfizierendes Mittel in Mengen von 0,0001 bis 1 Gew.-%, bezogen auf die Zusammensetzung, und
(f) mindestens ein Additiv in Mengen von 0,01 bis 10 Gew.-%, bezogen auf die Zusammensetzung,
enthält.

17. Behältnis, insbesondere Pumpspende oder Tube, aufweisend eine Zusammensetzung nach einem der Ansprüche 1 bis 16.
